# EUROPÄISCHE PATENTSCHRIFT

(11) **EP 0 707 211 B1**
(45) Veröffentlichungstag und Bekanntmachung des Hinweises auf die Patenterteilung: **28.07.1999**
(21) Anmeldenummer: 95114596.0
(22) Anmeldetag: 11.10.1995
(51) Int. Cl.: G01N 33/543, G01N 33/553

(54) **Regenerierbare Festphase zur Durchführung spezifischer Bindereaktionen**
Reusable solid phase for specific binding reactions
Phase solide réutilisable pour les réactions des liaisons spécifiques

(30) Priorität: 15.10.1994 DE 4436910
(43) Veröffentlichungstag der Anmeldung: 17.04.1996
(73) Patentinhaber: Dade Behring Marburg GmbH, 35001 Marburg (DE)
(72) Erfinder: Wiegand, Andreas, D-34613 Schwalmstadt (DE); Schmidtberger, Rudolf, D-35041 Marburg (DE)

(56) Entgegenhaltungen:
- EP-A- 0 524 663
- WO-A-92/02818
- DE-A- 2 433 246

## Beschreibung

Die vorliegende Erfindung betrifft ein Verfahren zur Durchführung immunchemischer Reaktionen sowie dafür speziell verwendbare regenerierbare Festphasen.

Immunchemische Nachweisverfahren haben in der in vitro Diagnostik eine hohe Bedeutung erlangt. Ursache dafür ist, daß sie hochspezifisch und äußerst empfindlich sind. Zudem zeichnen sich diese Verfahren durch eine einfache Handhabung aus.

Die Nachweisverfahren beruhen auf der immunologischen Wechselwirkung zwischen dem nachzuweisenden Analyten und seinem Bindungspartner bzw. seinen Bindungspartnern.

Generell werden immunologische Reaktionen in zwei Klassen eingeteilt: in homogene Verfahren und heterogene Verfahren. Diese zwei Verfahren unterscheiden sich unter anderem dadurch, daß bei den heterogenen Verfahren ein Reaktionspartner im Überschuß angeboten wird und dieser Überschuß vom Reaktionsprodukt abgetrennt werden muß. Bei den als homogenen Assays bekannten Verfahren ist eine solche Trennung nicht erforderlich, da sich die Signale der freien und gebundenen Markierung unterscheiden.

Immunchemische Verfahren und die verschiedenen Ausführungsformen sind dem Fachmann grundsätzlich bekannt.

Eine in der Proteindiagnostik weit verbreitete Methode die "bound/free" Trennung durchzuführen, besteht darin, einen Reaktionspartner adsorptiv oder kovalent an eine Festphase zu binden. Diese als "enzyme-linked immunosorbent assay" (ELISA) bekannte Technik wird in verschiedenen Varianten zum Nachweis von Antigenen bzw. Antikörpern verwendet.

Seit dem Bekanntwerden dieser Nachweistechnik sind immer wieder Versuche angestellt worden, die Festphase mit dem an sie gebundenen spezifischen Bindungspartner nach Ablauf der Reaktion zu regenerieren, z. B. dadurch, daß die während der Reaktion entstandenen Antigen-Antikörperkomplexe dissoziiert werden sollten.

Die für eine solche Dissoziation in Frage kommenden Agenzien, wie verdünnte Salz- oder Schwefelsäure, organische Säuren wie Essig- oder Propionsäure, konzentrierte Harnstofflösungen sowie Lösungen von Chaotropika wie KJ oder KSCN sind aber auch als denaturierend bekannt. Nachteilig kommt hinzu, daß die Dissoziation eines Antigen-Antikörpermittels dieser Reagenzien nur selten vollständig und im Hinblick auf eine Wiederverwendung nach einem unakzeptabel langen Zeitraum erfolgt.

Die bisher bekannten Verfahren den Festphasen-gebundenen spezifischen Bindungspartner zu regenerieren, sind trotz großer Fortschritte mit dem Nachteil behaftet, daß bei jedem Regenerierungsschritt in nicht bekanntem Ausmaß die Fesphase in ihren Bindungseigenschaften verändert wurde, dadurch bedingt haben bisher diese Verfahren keine kommerzielle Verwendung gefunden. (Siehe z.B. DE 2 433 246, EP 524663 oder WO 9 202 818)

Der vorliegenden Erfindung lag somit das technische Problem zugrunde, ein Verfahren bereit zu stellen, wodurch eine Festphase zur Immobilisierung spezifischer Bindungspartner mehrfach reproduzierbar in einem immunologischen

Nachweisverfahren eingesetzt werden kann. Die Lösung dieses technischen Problems erfolgt durch die Bereitstellung der in den Patentansprüchen gekennzeichneten Ausführungsformen.

Es wurde überraschenderweise festgestellt, daß bei der Verwendung von Edelmetallen, vorzugsweise Gold als Festphase sowie bestimmter Reduktions- oder Oxidationsmittel, wie z. B. Natriumborhydrid, Tetrabutylammoniumhydroxid mit oder ohne Zusatz von Detergenzien, die Festphase nach Regeneration mehrfach eingesetzt werden kann.

Bei dem erfindungsgemäßen Verfahren wird an einer Edelmetalloberfläche ein erster spezifischer Bindungspartner immobilisiert. Nach Waschung der beschichteten Oberfläche wird der Analyt mit dem immobilisierten spezifischen Bindungspartner zur Wechselwirkung gebracht und ggf. erneut gewaschen. In einem folgenden Schritt wird der immobilisierte Analyt mit einem markierten, zweiten spezifischen Bindungspartner zur Reaktion gebracht. Ggf. nach einem weiteren Waschschritt ist nun möglich, entweder direkt oder mittels einer weiteren Reaktion, eine dem Analyten äquivalente physikalische oder chemische Größe zu messen. Im folgenden Regenerationsschritt wird die Edelmetalloberfläche mittels eines wie oben beschriebenen Reagenzes von den anhaftenden Molekülen befreit und steht somit einer erneuten Beschichtung mit einem ersten spezifischen Bindungspartner zur Verfügung.

Die Erfindung eignet sich insbesondere zum Einbau in dem Fachmann an sich bekannte Geräte zu Einsatz in der Diagnostik. Vorteilhaft sind auch solche Ausführungen des Erfindungsgegenstandes, bei denen auf einen, für das Meßsignal durchlässigen Grundkörper eine für das Meßsignal ebenfalls durchlässige Edelmetallschicht angebracht wird, wobei die Signalabschwächung auch bei Kombination von Grundkörper und Edelmetallschicht bis zu 90 %, bevorzugterweise bis zu 50 %, ganz bevorzugterweise bis zu 20 % betragen kann.

Die Vorteile des erfindungsgemäßen Verfahrens liegen im wesentlichen darin, daß nicht - wie bei dem bisherigen Stand der Technik - für jedes Experiment eine neue Festphase benötigt wird, sondern die Festphase beliebig oft eingesetzt werden kann. Bei dem erfindungsgemäßen Verfahren entstehen viel weniger Abfallprodukte als bei dem gegenwärtigen Stand der Technik. Das erfindungsgemäße Verfahren erlaubt es, dem Anwender nach dem sogenannten "random excess Prinzip" vorzugehen, d. h. es ist möglich, Experimente mit verschiedenen ersten spezifischen Bindungspartnern hintereinander durchzuführen, ohne dabei die Festphase wechseln zu müssen.

Die folgenden Beispiele erläutern die Erfindung.

### Beispiel 1

### Quantitative human IgE-Bestimmung nach dem Sandwich-Prinzip mit chemilumineszenz-markierten Antikörpern

Testdurchführung und Regenerierung erfolgten bei einer Temperatur von +37 °C.
- Träger:: Rohr aus Feingold, Reinheit 99,99 % der Fa. DEGUSSA,
Außendurchmesser: 1,5 mm
Innendurchmesser: 0,7 mm
Länge 200 mm

### Beschichtung:

In das Rohr wurden 77 µl Lösung eines monoklonalen Antikörpers (Maus) gegen human IgE (MAK) gesaugt. Die Konzentration des MAK betrug 50 µg/ml. Die Lösung ent- hielt außerdem 75 mM Na-Phosphat, 75 mM NaCl, 100 g/l Na₂SO₄. Der pH-wert war 6,0. Die MAK-Lösung wurde 5 Minuten in dem Rohr belassen.

### Waschschritt:

Das Rohr wurde anschließend mit 1 ml Waschpuffer, d. h. einer Lösung aus 50 mM Tris(hydroxymethyl)aminomethan (TRIS) und 50 mM Zitronensäure, pH 7,4 gespült. Spüldauer 1 Minute.

### Probeninkubation:

77 µl definierte Mengen an IgE enthaltendes Humanserum wurde in das Rohr gesaugt und 10 Minuten darin belassen.

### Waschschritt:

Das Rohr wurde anschließend mit 1 ml Waschpuffer (5 mM Na-Phosphat, 85 mM NaCl, 1 g/l Tween 20®, 0,5 g/l Phenol, pH 6,5) 1 Minute lang gespült.

### Konjugat-Reaktion:

77 µl Lösung eines Konjugates (Herstellung analog Beispiel 8 aus EP-A 0 330 050) von polyklonalen Antikörpern (Kaninchen) gegen human-IgE mit Acridiniumester wurden in das Rohr gesaugt und 5 Minuten darin belassen.

Die Konzentration des Konjugates war 190 ng/ml, neben 0,1 M Na-Phosphat, 0,15 M NaCl, 1 g/l h-Serumalbumin, 1,5 g/l Tego-Betain L7®, pH 6,0.

### Waschschritt:

Das Rohr wurde anschließend innerhalb 1 Minute mit 1 ml einer Lösung von 75 mM Na-Phosphat und 75 mM NaCl, pH 7,2 gespült.

### Abspaltung des gebundenen Tracers.

Die Abspaltung des Tracers (Acridiniumester) erfolgt mit 77 µl einer Lösung von 0,1 m HNO₃ und 5 g/l H₂O₂ bei einer Inkubationszeit von 1 Minute.

### Messung:

Der den abgespaltenen Tracer enthaltende Rohrinhalt wurde in die Meßküvette des Luminometers AUTOCLINILUMAT, Fa. BERTHOLD, überführt und durch Zugabe von 300 µl 0,25 m NaOH die Lumineszenz-Reaktion ausgelöst.

### Regenerierung der Festphase:

Die Regenerierung erfolgt durch Einwirkung von 77 µl einer 10 g/l NaBH₄ enthaltenden Lösung, welche vorzugsweise zwischen pH 7,0 und 10,0 gepuffert ist (z.B. CHES), während 1 Minute und nachfolgendem Spülen mit 1 ml einer Lösung von 75 mM Na-Phosphat und 75 mM NaCl, pH 7,2. Spüldauer 1 Minute.

Vier, nach der oben beschriebenen Methode getestete Proben mit unterschiedlichem IgE-Gehalt ergeben das in der nachfolgenden Abbildung wiedergegebene Verhältnis Signalhöhe zu IgE-Gehalt. Letzteres ist in internationalen Einheiten angegeben.

### Beispiel 2

### Quantitative POD-Bestimmung

Testdurchführung und Regenerierung erfolgten bei einer Temperatur von +37 °C.
- Träger:: Rohr aus Feingold, Reinheit 99,99 % der Fa. DEGUSSA,
Außendurchmesser: 1,5 mm
Innendurchmesser: 0,7 mm
Länge 200 mm

### Beschichtung:

In das Rohr wurden 77 µl Lösung eines monoklonalen Antikörpers (Maus) gegen POD in PBS (pH 7,2) gesaügt. Nach 10minütiger Inkubationszeit wurde die Kapillare mit 2 ml einer 50 mmol TRIS-Citratpufferlösung (pH 7,4) gespült.

### Immunreaktion:

In die Goldkapillare wurden 77 µl einer Peroxidaselösung in IgE-Inkubationsmedium (Behringwerke AG, Marburg, Produkt Nr. OSND - 50 mmol Phosphat, 150 mmol NACl, 5 mmol Titriplex III, 0,1 % RSA, 0,01 % R-IgG, 10 % Glycerin, 2 % Tween 20, 2 mmol Phenol, pH 6,8)) gesaugt. Nach 10minütiger Inkubation wurde die Kapillare mit 2 ml Waschlösung POD, Behringwerke AG, Marburg, Produkt Nr. OSEW (5 mmol Phosphat, 85 mmol NaCl, 0.1 % Tween 20, 0,005 % Phenol, pH 6,5) gespült.

### Substratreaktion:

In das Rohr wurden 77 µl Chromogen POD, Behring, Marburg, Produkt Nr. OWEY gelöst in Puffer POD, Behring, Marburg, Produkt Nr. OWEZ gesaugt. Nach 5minütiger Inkubationszeit wurden 50 µl der in der Kapillare enthaltenen Lösung entnommen.

### Stoppung:

Die der Kapillare entnommenen 50 µl wurden mit 50 µl Stopplösung POD (Behring werke AG, Marburg, Produkt Nr. OSFA) versetzt.

### Detektion:

Extinktionsmessung der erhaltenen Lösung in einem Photometer bei 492 nm.

### Regenerierung der Festphase:

Die Goldkapillare wurde mit 5 ml einer 20 % Gew-% Tetrabutylammoniumhydroxidlösung gespült. Anschließend wurde die Kapillare mit 1 ml einer phosphatgepufferen Kochsalzlösunge (pH 7,2) gespült.

## Patentansprüche

1. Verfahren zur Durchführung heterogener immunchemischer Reaktionen unter Verwendung einer regenerierbaren Festphase aus einem Edelmetall, bei dem ein erster spezifischer Bindungspartner reversibel direkt an die Festphase gebunden wird und nach erfolgter Reaktion die Festphase dadurch regeneriert wird, daß der erste spezifische Bindungspartner mittels eines Reagenz, das ein Reduktions- oder Oxidationsmittel enthält, von der Festphase abgelöst wird.

2. Verfahren nach Anspruch 1, wobei die Festphase rohrförmig ist.

3. Verfahren nach mindestens einem der Ansprüche 1 bis 2, wobei die Festphase ein mit dem Edelmetall beschichteter Träger ist.

4. Verfahren nach mindestens einem der Ansprüche 1 bis 3, wobei der erste spezifische Bindungspartner ein Immunglobulin ist.

5. Verfahren nach mindestens einem der Ansprüche 1 bis 4, wobei das Reagenz NaBH₄ enthält.

6. Verfahren nach mindestens einem der Ansprüche 1 bis 2, wobei das Reagenz Tetrabutylammoniumhydroxid enthält.

7. Verfahren nach einem der Ansprüche 1 - 6, welches folgende Schritte beinhaltet:
a) Beschichten des Trägers mit einem ersten spezifischen Bindungspartner
b) Probeninkubation mit dem beschichteten Träger
c) Konjugatreaktion des Konjugats mit den vorhandenen immobilisierten Analyten
d) Messung der den Analyten äquivalenten physikalischen oder chemischen Größe
e) Regenerieren der Festsphasenoberfläche nach einem Verfahren gemäß einem der Ansprüche 5 - 6.

8. Verwendung einer regenerierbaren Festphase aus einem Edelmetall in einem Verfahren nach Anspruch 1.

## Claims

1. A method for carrying out heterogeneous immunochemical reactions using a regenerable solid phase made of a precious metal, in which a first specific binding partner is bound directly to the solid phase in a reversible manner and, after the reaction is complete, the solid phase is regenerated by the first specific binding partner being removed from the solid phase by means of a reagent which contains a reducing or oxidizing agent.

2. The method as claimed in claim 1, wherein the solid phase is tubular.

3. The method as claimed in at least one of claims 1 to 2, wherein the solid phase is a support which is coated with the precious metal.

4. The method as claimed in at least one of claims 1 to 3, wherein the first specific binding partner is an immunoglobulin.

5. The method as claimed in at least one of claims 1 to 4, wherein the reagent contains NaBH₄.

6. The method as claimed in at least one of claims 1 to 2, wherein the reagent contains tetrabutylammonium hydroxide.

7. The method as claimed in one of claims 1 - 6, which method comprises the following steps:
a) coating the support with a first specific binding partner,
b) incubating the sample with the coated support,
c) reacting the conjugate with the immobilized analytes which are present,
d) measuring the physical or chemical quantity which is equivalent to the analytes,
e) regenerating the solid phase surface by means of a method as claimed in one of claims 5 - 6.

8. The use of a regenerable solid phase made of a precious metal in a method as claimed in claim 1.

## Revendications

1. Procédé pour l'exécution de réactions immuno-chimiques hétérogènes, avec utilisation d'une phase solide à base d'un métal noble, pouvant être régénérée, dans lequel un premier partenaire de liaison spécifique est réversiblement fixé directement à la phase solide et, une fois effectuée la réaction, la phase solide est régénérée par le fait que le premier partenaire de liaison spécifique est détaché de la phase solide au moyen d'un réactif qui contient un réducteur ou un oxydant.

2. Procédé selon la revendication 1, dans lequel la phase solide est tubulaire.

3. Procédé selon au moins l'une des revendications 1 et 2, dans lequel la phase solide est un support revêtu avec un métal noble.

4. Procédé selon au moins l'une des revendications 1 à 3, dans lequel le premier partenaire de liaison spécifique est une immunoglobuline.

5. Procédé selon au moins l'une des revendications 1 à 4, dans lequel le réactif contient NaBH₄.

6. Procédé selon au moins l'une des revendications 1 et 2, dans lequel le réactif contient de l'hydroxyde de tétrabutylammonium.

7. Procédé selon l'une des revendications 1 à 6, qui comporte les étapes suivantes:
a) revêtement du support avec un premier partenaire de liaison spécifique,
b) incubation de l'échantillon avec le support revêtu,
c) réaction du conjugué avec l'analyte immobilisé présent,
d) mesure de la grandeur physique ou chimique équivalente à l'analyte,
e) régénération de la surface de la phase solide selon un procédé conforme à l'une des revendications 5 et 6.

8. Utilisation d'une phase solide à base d'un métal noble, pouvant être régénérée, dans un procédé selon la revendication 1.
